# EUROPEAN PATENT APPLICATION

(11) **EP 2 335 670 A1**
(43) Date of publication of application: **22.06.2011**
(21) Application number: 10190733.5
(22) Date of filing: 10.11.2010
(51) Int. Cl.: A61K 8/02, A61K 8/34, A61K 8/39, A61K 8/41, A61K 8/44, A61K 8/46, A61K 8/49, A61K 8/60, A61K 8/65, A61K 8/73, A61K 8/86, A61K 8/88, A61Q 5/06

(54) **A hair building solid**

(30) Priority: 27.11.2009 GB 0920779
(71) Applicant: Pangaea Laboratories Limited, London NW2 2AD (GB)
(72) Inventor: Isaacs, Elliot, London, NW2 2AD (GB)
(74) Representative: Hands, Lewis Roger

(57) **Abstract**

A hair building solid comprising a coating, wherein the coating facilitates the formation of a dipolar electrostatic charge on the surface of the coated solid.
The solid may be keratin, nylon or rayon and suitable coatings include surfactants. The solid and/or coating may comprise a humectant and/or hygroscopic substance, a conductive material, a UV protective material or a conditioning or emollient agent. The solid may have a least one tapered and/or bifurcated portion and/or concave portion on its surface. The solid may be a powder or fibre used to thicken hair or disguise hair loss formation and maintenance of electrostatic charge to adhere solid to hair and skin is improved.

## Description

The present invention relates generally to a hair building solid and to a method of applying such a hair building solid.

Hair building solids may take different forms. However, typically they are powders and/or fibres. The fibres are relatively narrow (typically less than 200 nm in diameter) and short (typically less than 0.7 mm in length). They typically comprise keratin, rayon or nylon. The solids may be coloured to match a user's hair. The solids are applied to the scalp and/or the hairs of the user typically in an area where the user has suffered hair loss. The solids adhere to the hairs and/or scalp and in the case of the former help to thicken the hairs appearance, and in the case of the latter to camouflage the skin.

The adherence of the solids to the scalp/hairs is typically enabled by means of electrostatic charge, although other means such as adhesives (for example hair spray) may be used. The solids are typically non-conductive and thus an electrostatic charge may be built up on their surface. This build-up may be due to contact with other surfaces, such as other solids, or the container in which the solids are stored. The built-up charge may last a relatively long time on the surface of the solids until such time as they are discharged to a neutral or oppositely charged surface.

The present invention describes a hair building solid wherein the formation and maintenance of an electrostatic charge on at least a portion of its surface is improved and/or enhanced. In this regard, the coating may allow greater charge to be built-up, and/or to facilitate a longer life of a charge before it decays, and/or to allow different charges on different portions of the solid, and/or to allow the formation of a charge in a less onerous manner.

This enhancement may be due to the different charging potential provided by differing materials according to their relative position in the triboelectric series. In this sense, the "materials" may be the solid together with the coating, or may be just the coating.

The invention provides hair building solids which predominantly do not lie flat against the user's hair or skin in use. Rather they lie with orientations to the user's hair and/or skin so as to promote the thickening and/or camouflage discussed above.

In a first aspect, the invention provides a hair building solid comprising a coating, wherein the coating facilitates the formation of a dipolar electrostatic charge on the surface of the coated solid.

In this regard, the term dipolar is taken to mean that there are two charges, of opposite sign or polarity, separated by a distance. There may be only two areas of charge on the solid. The magnitude of the two charges may or may not be substantially equal in magnitude.

The solid may comprise one or more of a polymer, keratin, nylon, a chelating agent and rayon.

The solid may at least partially comprise material which produces a positive or negative charge due to the triboelectric effect.

The coating may comprise one or more of an anionic, a non-ionic and a cationic surfactant. The anionic surfactant may comprise one or more of sodium lauryl sulphate, sodium laureth sulphate, sodium alkyl sulphate and sodium lauroyl sarcosinate. The cationic surfactant may comprise one or more of cetearyl alcohol, stearyl alcohol, cetyl alcohol and derivatives of quaternary ammonium compounds. The non-ionic surfactant may comprise one or more of decyl glucoside, coco glucoside, MEA, DEA, glycereth-2-2 cocoate; polysorbate 20, polysorbate 40, polysorbate 60, polysorbate 80, polysorbate 120; lauryl glycoside.

The coating may further comprise an amphoteric surfactant. The amphoteric surfactant may comprise one or more of cocamidopropylbetaine, dodecylbetaine, cocoamphoglycinate, oleyl betaine and soyamidopropylbetaine.

The solid may comprise two or more sequentially applied coatings.

The solid may comprise one or more chelating agents. Such a chelating agent is EDTA.

Either or both of the solid and the coating may comprise a humectant and/or a hygroscopic substance. The humectant and/or hygroscopic substance may comprise any one or more of allantoin, a form of hyaluronic acid, panthenol, sorbitol, glycerin, and sodium hyaluronate. Humectants and/or hygroscopic substances may increase the water content of the solid and/or coating which would, in turn, effect its conductivity and thus the potential for the formation, maintenance, and loss of an electrostatic charge.

The solid may comprise a conductive material. The conductive material may be one or more of a metal, a material formed of molecules rich in metal ions, a material with significant water and/or alcohol content, and a dye.

Conductive material may increase the conductivity of the solid and/or coating and thus the potential for the formation, maintenance, and loss of an electrostatic charge.

Either or both of the solid and the coating may comprise a UV protective material. The material may be any one or more of propanediol, quaternium-95, polyacrylate-15, titanium dioxide, zinc oxide, ethylhexyl methoxycinnamate, butyl methoxydibenzoylmethane, octyl methoxycinnamate, menthyl anthranilate, homosalate, benzophenone-3 and benzophenone-4.

Either or both of the solid and the coating may comprise a conditioning or emollient agent. The conditioning or emollient agent may be one or more of a silicone, a ceramide, coconut oil, a polyquaternium conditioner, ceramide 1, polyquaternium 22, and polyquaternium 19.

The solid may have at least one tapered portion and/or at least one bifurcated portion, and/or at least one concave portion on its outer surface.

The shape of the solid may be substantially linear, or may be substantially rectilinear, or may be substantially arcuate. In one embodiment, the solid may have one or more portions each portion having one of the three above described shapes.

In this regard, relatively long, narrow solids provide substantially well spaced portions each of which may accommodate an electrostatic charge, the polarity of each portion possibly being either negative or positive. For instance, a negative charge may be built-up at a first end and a positive charge at a second opposite end longitudinally spaced from the first end. Alternatively, a charge may only be built-up at one end, so that in use, that end may be attracted to a user's hair and/or skin allowing the other end to extend away from the hair and/or skin thus providing the desired thickening and/or camouflage effect.

Arcuate solids may promote electrostatic charge build-up in certain areas such as the concave or convex portions and/or at the ends. This may allow the solids to be attracted to the user's hair and/or skin in certain orientations. This may prevent , or at least slow down, the "earthing" of the solids whereby the charge(s) are lost.

A tapered end may increase the density and/or decrease the magnitude of an electrostatic charge built-up thereon. It is possible that a tapered end spreads an electrostatic charge along a greater length of the sides of the solid as compared to a solid without a tapered end.

A solid having a concave portion in the surface of a solid will have a greater surface area than one without and thus has the potential to carry a greater amount of electrostatic charge.

The various shapes discussed above may also promote collisions between individual solids. Alternatively, or additionally, they may promote collisions between the solids and/or a dispenser before and/or during application so that together with the coating more useful charged portions are formed and/or maintained on the surface of the solids. The usefulness of the charge(s) may be due to any one or more of their magnitude, number of discrete charged areas, polarity and mix of polarities on the same solid.

The solid may have a length in the range 95µm to 50mm, although other lengths are contemplated. The length may need to be sufficient to allow the formation and retention of independent charges at either end of the solid.

The coatings may form a "reservoir" of variously charged and/or chargeable materials. This effect may be known as "electrostatic buffering".

In one embodiment, the invention provides a method of applying a solid, as described and/or as claimed herein, to hair and/or skin, the method including the step of providing a device which affects or maintains an electrostatic charge on said hair, skin or solid. The device may be a hair building solid dispenser, a wand, brush, roller or comb that is electrostatically charged or made of a material that produces charge due to the triboelectric effect. The device may have another primary purpose such as for styling the hair, and/or treating the hair and/or scalp.

The method may include the step of applying an amphoteric surfactant and/or dipolar amino acids.

The method may include the step of applying one or more alcohol soluble polymers such as acrylates, octylacrylamide copolymer, C12-22 alkylmethacrylate copolymer, polyurethane-14 AMP-acrylates copolymer, PVP, dimethylaminoethylmethacrylate copolymer, polyquaternium 69, isobutylene ethylmaleimide copolymer, isobutylene hydroxyethylmaleimide copolymer and polymethylsilsesquioxane.

Whilst the embodiments described herein are related to fibres, it is also possible to utilise any of the described technologies or methods in association with any other type of solid, such as a powder, or even a liquid droplet such as is emitted from an aerosol in certain hair building sprays well known to those skilled in the art.

It is also possible that some methods of manufacture and packaging may increase the potential for hair building products to obtain dipolar charges. Tools used during the manufacturing process may comprise materials that impart electrostatic charge due to the triboelectric effect, or alternatively may be conductive in order to prevent the build-up of any unwanted charge. Similarly the packaging of the hair building solids could comprise the materials discussed above, or even contain ionised air to increase the products' tendency to gain electrostatic charge.

The above and other characteristics, features and advantages of the present invention will become apparent from the following detailed description, taken in conjunction with the accompanying drawings, which illustrate, by way of example, the principles of the invention. This description is given for the sake of example only, without limiting the scope of the invention. The reference figures quoted below refer to the attached drawings.

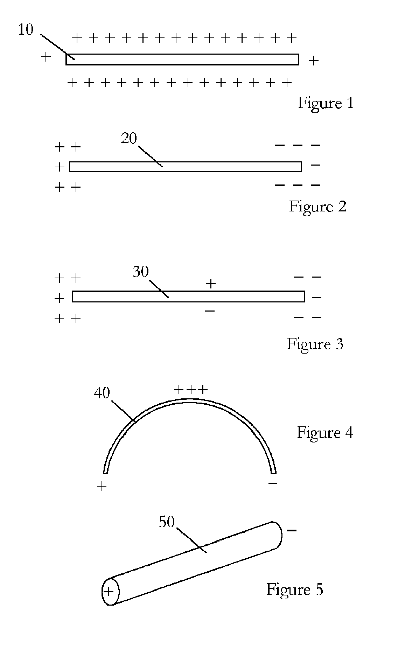

Figures 1 to 14 show different embodiments of a hair building solid including various different arrangements of electrostatic charges on their surfaces.

The present invention will be described with respect to particular embodiments and with reference to certain drawings but the invention is not limited thereto but only by the claims. The drawings described are only schematic and are non-limiting. In the drawings, the size of some of the elements may be exaggerated and not drawn to scale for illustrative purposes. The dimensions and the relative dimensions do not correspond to actual reductions to practice of the invention.

Furthermore, the terms first, second, third and the like in the description and in the claims, are used for distinguishing between similar elements and not necessarily for describing a sequence, either temporally, spatially, in ranking or in any other manner. It is to be understood that the terms so used are interchangeable under appropriate circumstances and that the embodiments of the invention described herein are capable of operation in other sequences than described or illustrated herein.

Moreover, the terms top, bottom, over, under and the like in the description and the claims are used for descriptive purposes and not necessarily for describing relative positions. It is to be understood that the terms so used are interchangeable under appropriate circumstances and that the embodiments of the invention described herein are capable of operation in other orientations than described or illustrated herein.

It is to be noticed that the term "comprising", used in the claims, should not be interpreted as being restricted to the means listed thereafter; it does not exclude other elements or steps. It is thus to be interpreted as specifying the presence of the stated features, integers, steps or components as referred to, but does not preclude the presence or addition of one or more other features, integers, steps or components, or groups thereof. Thus, the scope of the expression "a device comprising means A and B" should not be limited to devices consisting only of components A and B. It means that with respect to the present invention, the only relevant components of the device are A and B.

Similarly, it is to be noticed that the term "connected", used in the description, should be interpreted as possibly meaning that two or more elements are either in direct physical or electrical contact, or that two or more elements are not in direct contact with each other but yet still co-operate or interact with each other.

Reference throughout this specification to "one embodiment" or "an embodiment" means that a particular feature, structure or characteristic described in connection with the embodiment is included in at least one embodiment of the present invention. Thus, appearances of the phrases "in one embodiment" or "in an embodiment" in various places throughout this specification are not necessarily all referring to the same embodiment, but may refer to different embodiments. Furthermore, the particular features, structures or characteristics may be combined in any suitable manner, as would be apparent to one of ordinary skill in the art from this disclosure, in one or more embodiments.

Similarly it should be appreciated that in the description of exemplary embodiments of the invention, various features of the invention are sometimes grouped together in a single embodiment, figure, or description thereof for the purpose of streamlining the disclosure and aiding in the understanding of one or more of the various inventive aspects. This method of disclosure, however, is not to be interpreted as reflecting an intention that the claimed invention requires more features than are expressly recited in each claim. Rather, as the following claims reflect, inventive aspects lie in less than all features of a single foregoing disclosed embodiment. Thus, the claims following the detailed description are hereby expressly incorporated into this detailed description, with each claim standing on its own as a separate embodiment of this invention.

Furthermore, while some embodiments described herein include some but not other features included in other embodiments, combinations of features of different embodiments are meant to be within the scope of the invention, and form different embodiments, as would be understood by those skilled in the art. For example, in the following claims, any of the claimed embodiments can be used in any combination.

In the description provided herein, numerous specific details are set forth. However, it is understood that embodiments of the invention may be practised without these specific details. In other instances, well-known methods, structures and techniques have not been shown in detail in order not to obscure an understanding of this description.

The invention will now be described by a detailed description of several embodiments of the invention. It is clear that other embodiments of the invention can be configured according to the knowledge of persons skilled in the art without departing from the true spirit or technical teaching of the invention, the invention being limited only by the terms of the appended claims.

In the following examples, the electrostatic charges are predominantly shown as being arranged around, or at, the ends of the hair building solids. This is due to the nature of electrostatic charges collecting, or being concentrated, at such places. However, it should be understood that the arrangements shown are not limiting as electrostatic charges will also be formed or maintained, in some embodiments, in other places either as well as, or instead of, at the ends.

Although no individual coatings, as such, are represented, it should be understood that the solids depicted may include one or more such coatings on all or at least a portion of their surface. Such coatings may penetrate the surface of the solids.

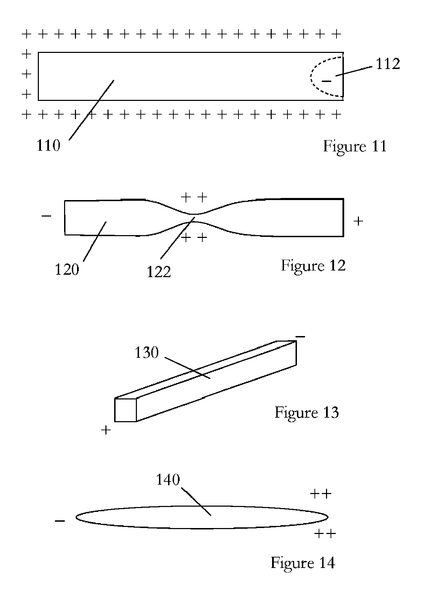

Figure 1 show a hair building solid in the form of an essentially flat rectangle, such as a length of tape. Alternatively, the solid 10 shown in Figure 1 could be one side of a substantially rectangular box-shape such as shown in Figure 13, or one side of a tube or cylinder as shown in Figure 5. An electrostatic charge is arranged substantially across the entire surface of the solid having a positive polarity.

Alternatively, the solid 20 may have a dipolar charge as shown in Figure 2, such that there is a negative charge at one end of the solid and a positive charge at the other opposite end.

Another alternative, depicted in Figure 3, is that the solid 30 has a dipolar charge in that an electrostatic charge is arranged at either end having opposite polarities from each other but also including two areas of intermediate charge, arranged approximately midway along the longitudinal length of the solid, separate from the charges at the ends of the solid. These intermediate areas have opposite polarities from each other.

The solid 40 in Figure 4 is substantially arcuate in form having a positive charge at one end, a negative charge at the other opposite end and a discrete positive charge arranged at an approximate mid-point along one side. The form of the solid 40 is substantially semi-circular.

The cylinder or tube 50 shown in Figure 5 has a positive charge at one longitudinal end and a negative charge at the other opposite end.

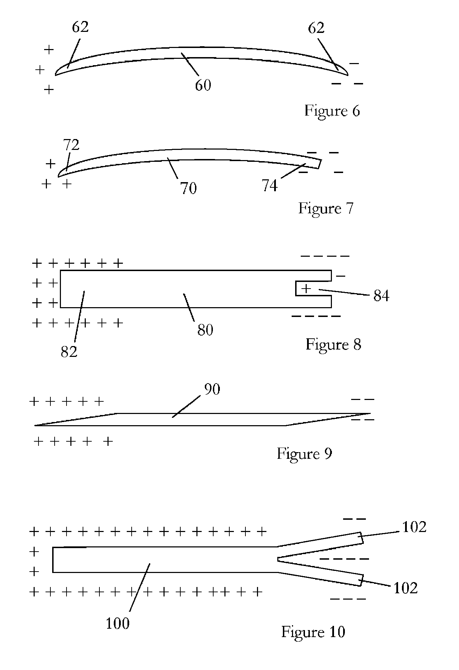

Figure 6 shows another arcuate form of solid 60, however, this one is more crescent in shape than the one shown in Figure 4. It has two substantially tapered ends 62 where the electrostatic charge is provided, one being positive and the other negative. A similar solid 70 is shown in Figure 7, however, it has only one tapered end 72, the other, opposite, end 74 being blunt. Again, a positive charge is provided at one end (the tapered end 72 in this Figure) and a negative charge at the other (blunt) end 74.

It is also possible to produce solids 80 having a notch 84 in one end, the other longitudinal end 82 being without such a notch 84, although it is possible for notches to be provided in both ends. In this embodiment, a charge of one polarity may be provided within the notch 84 while a charge of another polarity may be provided on the surface surrounding the notch 84. A charge may be provided at the other longitudinal end 82 of the solid 80 which is shown as being positive.

Figure 9 shows how solid 90 may be rhomboid in shape. The view in Figure 9 may be of a substantially laminar-like material, such as a tape, or the side view of a solid 90 having a thickness, which although not shown, may be of substantial dimension such that the solid would not be considered laminar-like, but would be more fibre-like. Again, a dipolar charge is shown with a positive charge at one end and a negative charge at the other end.

Some solids 100 may be bifurcated. This may occur at only end, as depicted in Figure 10, at both ends (not shown). The bifurcation may comprise of two tines 102. A charge may be present on the tines 102 and therebetween. These charges may be the same polarity or may have different polarities. In the example shown the tines 102 both have a negative charge, however, the charge shown along the sides of the solid away from the bifurcated end has a positive charge.

Another possibility is that the solid 110 may include a concave portion 112. This may be provided in one end of the solid or may be provided on the longitudinal side of the solid. A charge of one polarity may be present within the concave portion 112 and a charge of another polarity may be present on the surface of the solid 110.

The concave portion 122 may be provided all the way around a solid so as to form a waisted portion such as is shown in Figure 12. The charge within the waisted portion 122 may be of one polarity and the charge at other portions of the surface of the solid 120 may be of another polarity. For instance, there may be a positive charge in the waisted portion 122 and a negative charge arranged at the extreme ends of the solid 120.

A solid 130 having a parallelepiped form is shown in Figure 13. By contrast, a substantially oval form of solid 140 is shown in Figure 14. This may be substantially laminar and tape-like or may be like a rugby ball which has been stretched in the axial direction. Both of these solids 130, 140 have a positive charge at one end and a negative charge at the other end.

Other shapes are contemplated.

In use, the electrostatically charged solids may be attracted to a user's hair and/or skin so that they are substantially retained in place. It is useful to be able to encourage the formation, polarity and distribution, of charges on the solids. This allows for a greater degree of control of the product in use and may allow for the tailoring of charge types on an individual basis as possibly dictated by hair types, environment and other such factors.

## Claims

1. A hair building solid comprising a coating, wherein the coating facilitates the formation of a dipolar electrostatic charge on the surface of the coated solid.

2. The solid of claim 1, wherein the solid comprises one or more of a polymer, keratin, nylon and rayon.

3. The solid of any preceding claim, wherein the solid at least partially comprises material which produces a positive charge due to the triboelectric effect.

4. The solid of any preceding claim, wherein the coating comprises one or more of an anionic, a non-ionic and a cationic surfactant.

5. The solid of claim 4, wherein the anionic surfactant comprises one or more of sodium lauryl sulphate, sodium laureth sulphate, sodium alkyl sulphate and sodium lauroyl sarcosinate; the non-ionic surfactant comprises one or more of decyl glucoside, coco glucoside, MEA, DEA, glycereth-2-2 cocoate; polysorbate 20, polysorbate 40, polysorbate 60, polysorbate 80, polysorbate 120; lauryl glycoside; and the cationic surfactant comprises one or more of cetearyl alcohol, stearyl alcohol, cetyl alcohol and derivatives of quaternary ammonium compounds.

6. The solid of any preceding claim, wherein the coating further comprises an amphoteric surfactant.

7. The solid of claim 6, wherein the amphoteric surfactant comprises one or more of cocamidopropylbetaine, dodecylbetaine, cocoamphoglycinate, oleyl betaine and soyamidopropylbetaine.

8. The solid of any preceding claim, comprising two or more sequentially applied coatings.

9. The solid of any preceding claim, wherein either or both of the solid and the coating comprise a humectant and/or a hygroscopic substance.

10. The solid of claim 9, wherein the humectant and/or hygroscopic substance comprises any one or more of allantoin, a form of hyaluronic acid, panthenol, sorbitol, glycerin, and sodium hyaluronate.

11. The solid of any preceding claim, comprising a conductive material.

12. The solid of claim 11, wherein the conductive material is one or more of a metal, a material formed of molecules rich in metal ions, a material with significant water and/or alcohol content, and a dye.

13. The solid of any preceding claim, wherein either or both of the solid and the coating comprise a UV protective material.

14. The solid of any preceding claim, wherein the solid has at least one tapered portion and/or at least one bifurcated portion, and/or at least one concave portion on its surface.

15. The solid of any preceding claim, wherein either or both of the solid and the coating comprise a conditioning or emollient agent.
